# EUROPEAN PATENT APPLICATION

(11) **EP 4 385 970 A1**
(43) Date of publication of application: **19.06.2024**
(21) Application number: 22213126.0
(22) Date of filing: 13.12.2022
(51) Int. Cl.: C05C 9/00

(54) **UREA-PRODUCING PLANT WITH LOW CARBON DIOXIDE EMISSIONS**

(71) Applicant: Yara International ASA, 0277 Oslo (NO)
(72) Inventor: PORRO, Lino Giovanni, 1040 Etterbeek (BE)
(74) Representative: De Clercq & Partners

(57) **Abstract**

The present disclosure provides a urea-producing plant with low greenhouse gas emissions, in particular low carbon dioxide emissions. The plant comprises a first high-pressure steam network, a device connected to the first high-pressure steam network, and one or more electric boilers configured to produce high-pressure steam and deliver this high-pressure steam to the first high-pressure steam network. The present disclosure also provides a method for producing urea with low greenhouse gas emissions.

## Description

### Field of the disclosure

The present disclosure is related to the field of urea production.

### Background

Urea (H₂NCONH₂) is the most common nitrogen-containing fertilizer used today. Global annual production is estimated to be around 200 million tons. In addition to the use as fertilizer, urea is also used in various chemical synthesis, and as a solution in diesel-powered vehicles to reduce nitrogen oxides emissions.

Urea-producing plants may use different technologies, such as the Stamicarbon or Saipem process, but they all rely on the same basic principles. In the synthesis section of a urea-producing plant, ammonia (NH₃) and carbon dioxide (CO₂) are reacted under high pressure (above 130 bar) and high temperatures, to produce an aqueous solution comprising urea, ammonium carbamate ([NH₄][H₂NCO₂]), and free ammonia. This aqueous solution is then purified and concentrated to obtain a urea melt. The urea melt can be diluted to obtain a urea aqueous solution which may be used in selective catalytic or non-catalytic reduction systems, such as diesel exhaust fluid, mixed with other nutrient sources, such as ammonium nitrate, to obtain aqueous solutions of urea ammonium nitrate, or solidified into urea-containing solids, such as granules or prills.

The production of urea requires the input of high amounts of energy, in particular due to the reaction equilibrium existing between the starting materials and the final product. In most urea plants, this energy is obtained by burning non-renewable energy sources, such as natural gas or oil, to heat up water and to produce steam. However, this production of energy releases vast amounts of carbon dioxide, a greenhouse gas, into the atmosphere, and there is a constant requirement from authorities and clients to reduce emissions of carbon dioxide from urea production.

### Summary

A new method has now been identified to reduce carbon dioxide emissions in a urea plant and a urea plant has been designed with reduced carbon dioxide emissions.

It was found out that the boilers producing the high-pressure steam required by a urea-producing plant were one of the most important sources of greenhouse gas emissions, in particular CO₂, in a urea-producing plant. These boilers often use fossil fuels, such as natural gas, to heat up water and to produce steam. Replacing these boilers with electric boilers, thereby consuming electricity to produce heat, represents an important reduction in CO₂-emissions for the plant.

In a first aspect, the present disclosure provides a urea-producing plant comprising a first high-pressure steam network, a device connected to the first high-pressure steam network, and one or more electric boilers configured to produce high-pressure steam and deliver this high-pressure steam to the first high-pressure steam network.

In another aspect, the present disclosure provides a method for producing a urea melt in a urea-producing plant according to the present disclosure, wherein the urea-producing plant comprises a first high-pressure steam network, a device connected to the first high-pressure steam network, and one or more electric boilers, the method comprising the steps of:
a) producing high-pressure steam with the one or more electric boilers;
b) directing the high-pressure steam produced in step a) to the first high-pressure steam network;
c) mixing carbon dioxide and ammonia in a reactor, thereby producing a synthesis solution comprising water, urea, ammonium carbamate, and free ammonia;
d) providing high-pressure steam from the first high-pressure steam network and the synthesis solution produced in step c) to a high-pressure stripper, thereby producing an aqueous solution comprising urea and depleted of ammonium carbamate and free ammonia;
e) processing the aqueous solution obtained in step d) to obtain a urea melt.

### Brief description of the figures

The following description of the figures of a specific embodiment of a system according to the present disclosure is only given by way of example and is not intended to limit the present explanation, its application or use. In the figure, identical reference numerals refer to the same or similar parts and features.
Figure 1 represents the synthesis section of an embodiment of a urea-producing plant according to the present disclosure.
Figure 2 represents the synthesis section of another embodiment of a urea-producing plant according to the present disclosure.
Figure 3 represents the synthesis section of another embodiment of a urea-producing plant according to the present disclosure.
Figure 4 represents the synthesis section of another embodiment of a urea-producing plant according to the present disclosure.
Figure 5 represents a section of another embodiment of a urea-producing plant according to the present disclosure.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a device" refers to one or more than one device.

"Comprise", "comprising", and "comprises" and "comprised of" as used herein are synonymous with "include", "including", "includes" or "contain", "containing", "contains" and are inclusive or open-ended terms that specifies the presence of what follows, for example a component, and do not exclude or preclude the presence of additional, non-recited components, features, element, members, steps, known in the art or disclosed therein.

In a first aspect, the present disclosure provides a urea-producing plant comprising a first high-pressure steam network, a device connected to the first high-pressure steam network, and one or more electric boilers, configured to produce high-pressure steam and deliver this high-pressure steam to the first high-pressure steam network.

An electric boiler is a device configured to receive water, in particular demineralized water, and electricity, and to produce steam, which may be saturated or superheated. Electric boilers can be configured to produce steam at the pressure required by the plant.

In some embodiments, the one or more electric boilers are configured to produce steam at a pressure of 1.0 to 2.5 MPa.

The one or more electric boilers are connected to a first high-pressure steam network of the urea-producing plant, configured to receive high-pressure steam, and are configured to direct the high-pressure steam they produce to the first high-pressure steam network.

The first high-pressure steam network, hereafter the high-pressure (HP) steam network, is connected to devices that require high-pressure steam for operating. In some embodiments, the urea-producing plant comprises an HP stripper and/or a urea hydrolyzer. In some embodiments, the urea-producing plant comprises an HP stripper. In some embodiments, the urea-producing plant comprises a urea hydrolyzer. In some embodiments, the urea-producing plant comprises an HP stripper and a urea hydrolyzer.

An HP stripper is a device configured to receive a synthesis solution, which is an aqueous solution comprising urea, ammonium carbamate, and free ammonia, from the synthesis reactor and to heat up the synthesis solution with the help of HP steam, such that the ammonium carbamate decomposes back to ammonia and carbon dioxide. The gases are separated from the aqueous solution, such that an aqueous solution comprising urea, and being depleted of free ammonia and ammonium carbamate, is obtained. The aqueous solution produced by the HP stripper may still comprise some ammonium carbamate and free ammonia, but in a much smaller amount than the synthesis solution. An HP stripper also produces a gaseous stream comprising ammonia and carbon dioxide.

A urea hydrolyzer is a device configured to receive an aqueous solution comprising urea and to heat up said solution with the help of HP steam, such that the urea comprised in the solution is hydrolyzed into ammonia and carbon dioxide. A urea hydrolyzer produces an aqueous solution depleted of urea and a gaseous stream comprising ammonia and carbon dioxide. The aqueous solution produced by the hydrolyzer may be further processed to remove more ammonia, and/or carbon dioxide. In some embodiments, the hydrolyzer is connected to a desorber column, which is configured to remove ammonia and carbon dioxide from an aqueous solution.

The urea hydrolyzer may also be connected to the low-pressure (LP) section, such that the gas stream produced by the hydrolyzer can be re-injected in the process. The gas stream produced by the hydrolyzer may be injected as a gas stream in the LP section, or it may be first condensed as an aqueous solution and then injected in the LP section. In some embodiments, the urea hydrolyzer is connected to the LP section. In some embodiments, the urea hydrolyzer is connected to a condenser, and the condenser is connected to the LP section.

In some embodiments, the first high-pressure steam network is configured to receive steam at a pressure of from 1.0 to 11.0 MPa, from 2.0 to 11.0 MPa, from 2.0 to 10.0 MPa, from 2.0 to 9.0 MPa, from 2.0 to 8.0 MPa, from 2.0 to 7.0 MPa, from 2.0 to 6.0 MPa, from 2.0 to 5.0 MPa, from 2.0 to 4.0 MPa, from 2.0 to 3.0 MPa, from 2.0 to 2.9 MPa, from 2.0 to 2.7 MPa, from 2.0 to 2.6 MPa, from 2.0 to 2.5 MPa, from 2.0 to 2.4 MPa, from 1.5 to 2.9 MPa, from 1.5 to 2.7 MPa, from 1.5 to 2.6 MPa, from 1.5 to 2.5 MPa, or from 1.5 to 2.4 MPa, from 1.8 to 2.9 MPa, from 1.8 to 2.7 MPa, from 1.8 to 2.6 MPa, from 1.8 to 2.5 MPa, or from 1.8 to 2.4 MPa. The HP steam network may be configured to receive steam at a pressure specific to the plant. Urea plants use different technologies depending on which company built the plant. For example, in a Stamicarbon plant, i.e., a plant built by the technology provider Stamicarbon, the HP steam network is configured to receive steam at a pressure of from 1.8 to 2.5 MPa.

A urea-producing plant may also comprise a second steam network, in particular a second steam network configured to receive low-pressure (LP) steam.

In some embodiments, the second steam network is configured to receive steam at a pressure of from 0.1 to 0.7 MPa, from 0.2 to 0.7 MPa, from 0.1 to 0.6 MPa, from 0.2 to 0.6 MPa, from 0.3 to 0.6 MPa, or from 0.3 to 0.7 MPa. The second, LP, steam network may be configured to receive steam at a pressure specific to the plant.

A urea-producing plant comprises several devices that require LP steam to operate. Examples of devices that require LP steam include evaporators, carbamate decomposers, desorbers and steam ejectors.

A urea-producing plant does not require a specific device to produce low-pressure steam from water, such as a boiler. Instead, it uses steam produced by some devices comprised in the plant, in particular its HP carbamate condenser.

As mentioned above, a urea-producing plant comprises an HP stripper to remove free ammonia and ammonium carbamate from the synthesis solution. An HP stripper produces two streams: an aqueous solution comprising urea, and depleted of ammonium carbamate and free ammonia, and a gaseous stream comprising ammonia, carbon dioxide, and water. The components of the gaseous streams are valuable products, that can be recycled back into the synthesis reactor to produce more urea. However, the gaseous stream produced by the HP stripper contains more heat than that required by the urea reactor. So, re-injecting the gaseous stream from the HP stripper directly to the reactor would lead to a loss of energy. Instead, a urea-producing plant comprises a carbamate condenser, also called high-pressure (HP) carbamate condenser, that is configured to condensate the gaseous stream comprising carbon dioxide, ammonia, and water, produced by the HP stripper into an aqueous solution comprising ammonium carbamate and free ammonia. This aqueous solution can be injected into the reactor by use of an ejector, using ammonia as motive fluid or by gravity. Energy or heat from the gaseous stream produced by the HP stripper is recovered in the HP carbamate condenser in the form of steam, in particular low-pressure steam, that is directed to other devices comprised in the urea-producing plant.

In some embodiments, the plant comprises an HP carbamate condenser and an ejector, wherein the carbamate condenser is connected to the HP stripper and to the ejector, and the ejector is connected to the HP carbamate condenser and the synthesis reactor.

In some embodiments, the HP carbamate condenser is connected directly to the synthesis reactor and is configured to produce a two-phase stream (liquid and gas) which may be directly injected in the synthesis reactor without an ejector.

Condensing a gaseous stream comprising water, carbon dioxide, and ammonia, is an exothermic operation, and it is possible to recover the heat produced by the condensation by producing steam. A carbamate condenser is configured to condense a gaseous stream comprising ammonia, carbon dioxide and water, and produce steam. The steam produced by the carbamate condenser is LP steam, with a pressure of from 0.25 to 7.50 MPa, and is directed to the second steam network of the plant, such that it can be distributed to a device which requires low-pressure steam in operation.

The urea-producing plant comprises a synthesis section comprising a synthesis reactor, wherein ammonia and carbon dioxide are mixed under high pressure and high temperature to form an aqueous solution comprising urea and ammonium carbamate.

The synthesis reactor is connected to the HP stripper, such that the synthesis solution produced by the synthesis reactor can be sent to the HP stripper. The HP stripper removes some water, ammonium carbamate, and free ammonia, from the synthesis solution to produce a urea solution that still contains some ammonium carbamate and free ammonia but less than the synthesis solution.

The solution produced by the HP stripper is sent to a medium-pressure section or a low-pressure section. The medium-pressure section and/or the low-pressure section contain various equipment to further purify and concentrate the solution produced by the HP stripper. In some embodiments, the medium-pressure section and/or the low-pressure section contain any component selected from the group consisting of a condenser, a liquid-vapor separator, and a concentrator.

The low-pressure section produces a urea solution comprising at least 50.0 weight% of urea and from 0 to 50.0 weight% of water.

In some embodiments, the low-pressure section of the urea-producing plant is configured to produce a urea solution comprising from 50.0 to 80.0 weight%, from 60.0 to 80.0 weight%, from 50.0 to 75.0 weight%, from 60.0 to 75.0 weight%, from 65.0 to 80.0 weight%, from 65.0 to 75.0 weight%, or from 65.0 to 70.0 weight% of urea. In some embodiments, the low-pressure section of the urea-producing plant is configured to produce a urea solution comprising from 20.0 to 50.0 weight%, from 20.0 to 40.0 weight%, from 25.0 to 50.0 weight%, from 25.0 to 40.0 weight%, from 20.0 to 35.0 weight%, from 25.0 to 35.0 weight%, or from 30.0 to 35.0 weight% of water.

In some embodiments, the urea-producing plant comprises a concentration section. The concentration section is configured to receive a urea solution from the low-pressure section of the plant that may contain up to 50.0 weight%, up to 40.0 weight%, or up to 30.0 weight% of water, and produce a urea melt that may comprise at least 95.0 weight% of urea.

The concentration section may comprise one or more evaporators, and one or more liquid-vapor separators.

In some embodiments, the concentration section is connected to the solidification section of the plant. The solidification section is configured to a solid, particulate composition, for example via prilling or granulation.

Alternatively, the urea melt can also be diluted with water to produce a urea solution. This urea solution may be used as a diesel exhaust fluid (DEF).

The urea-producing plant may also comprise several loops to treat waste streams produced by some devices, recover ammonia and carbon dioxide from these waste streams, and send the recovered ammonia and carbon dioxide back to the synthesis section or the medium pressure or low-pressure section.

In some embodiments, the urea-producing plant comprises a carbon dioxide compressor and an electric motor configured to provide power to the carbon dioxide compressor. The synthesis of urea in the reactor is performed at high pressure (over 10 MPa), so a urea-producing plant comprises a carbon dioxide compressor to raise the pressure of the carbon dioxide feed. Such compressor requires an important amount of power to operate, and the plant comprises a power source connected to that compressor to provide this power. In a conventional urea-producing plant, this power source is often a gas or steam turbine. Such turbines are popular because they use a feed, gas or steam, that is readily available in the plant. However, a gas or steam turbine generates greenhouse gas emissions either directly for a gas turbine or indirectly for a steam turbine because steam is often produced by burning fossil fuels. So, to keep greenhouse gas emissions low, it is an advantage to use an electric motor to power the carbon dioxide compressor. Such power source can be considered as a low emitter of greenhouse gas as long as the electricity supplied to it is produced by methods not generating greenhouse gas emissions, such as solar, wind, sea, or nuclear power plants.

In some embodiments, the carbon dioxide compressor is connected to the synthesis reactor, and the carbon dioxide stream from the carbon dioxide compressor is configured to be injected directly in the synthesis reactor.

In some embodiments, the carbon dioxide compressor is connected to an HP stripper and the carbon dioxide stream from the carbon dioxide compressor is configured to be the HP stripper. Carbon dioxide may be used as a stripping gas in HP strippers to increase the transfer of gases from the aqueous solution to the gas phase. The gaseous stream produced by the HP stripper is eventually injected into the synthesis reactor after passing through the carbamate condenser. So, the carbon dioxide required for the synthesis of urea may be first used as a stripping gas before being injected in the synthesis reactor to form urea.

In some embodiments, the urea-producing plant comprises an electric motor configured to provide power to an ammonia pump. Ammonia is directed to the reactor under high pressure by one or more ammonia pumps. These pumps also require a power source to operate, and an electric motor ensures that this step does not contribute to greenhouse gas emissions.

In some embodiments, the ammonia pump is connected to an ejector configured to inject an aqueous solution into the synthesis reactor. Instead of injecting the ammonia alone in the synthesis reactor, it may be an advantage to direct the ammonia stream to an ejector which is configured to inject in the synthesis reactor an aqueous solution, in particular an aqueous solution produced by the HP carbamate decomposer. The ejector may use the ammonia stream as motive fluid. In such embodiments, the plant does not require another gas pump to inject the aqueous solution into the synthesis reactor.

In some embodiments, all the pumps comprised in the urea-producing plant are driven by electric motors. This ensures that the greenhouse gas emissions of the plant are kept as low as possible.

In some embodiments, the urea-producing plant comprises no direct sources of greenhouse gas emissions.

In another aspect, the present disclosure provides a method for producing a urea melt in a urea-producing plant according to the present disclosure, wherein the urea-producing plant comprises a first high-pressure steam network, a device connected to the first high-pressure steam network, and one or more electric boilers, the method comprising the steps of:
a) producing high-pressure steam with the one or more electric boilers;
b) directing the high-pressure steam produced in step a) to the first high-pressure steam network;
c) mixing carbon dioxide and ammonia in a reactor, thereby producing a synthesis solution comprising water, urea, ammonium carbamate, and free ammonia;
d) providing high-pressure steam from the first high-pressure steam network and the synthesis solution produced in step c) to a high-pressure stripper, thereby producing an aqueous solution comprising urea and depleted of ammonium carbamate and free ammonia;
e) processing the aqueous solution obtained in step d) to obtain a urea melt.

In some embodiments, the urea melt produced in step e) comprises at least 95 weight% of urea.

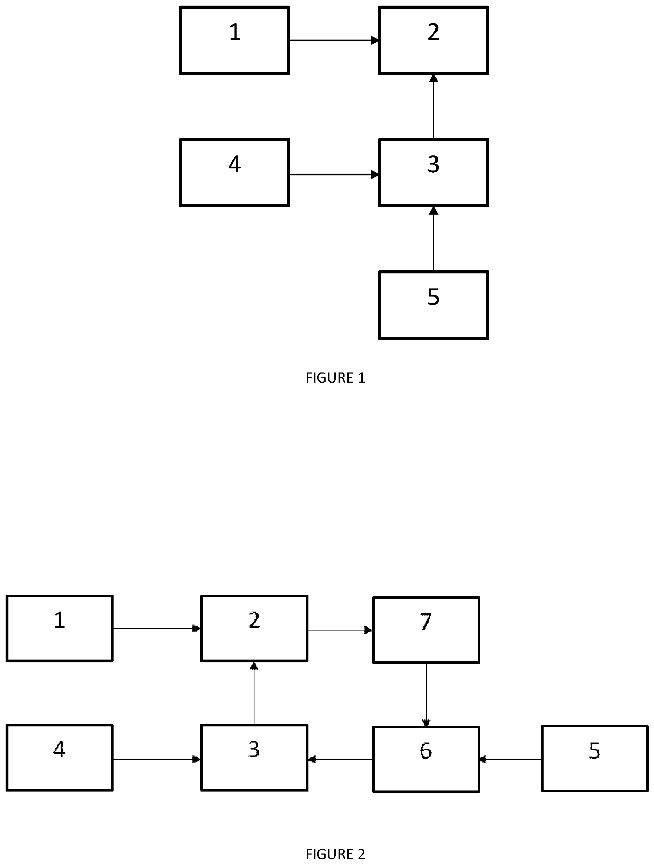

Figure 1 represents the synthesis section of an embodiment of a urea-producing plant according to the present disclosure. The plant comprises a boiling section **1** comprising one or more electric boilers depending on its steam requirement. The boilers are configured to produce high-pressure steam at a pressure of above 2.0 MPa or from 2.0 to 2.6 MPa. The boiling section is connected to the HP stripper **2** of the urea-producing plant via a first high-pressure steam network. The stripper **2** is connected to the synthesis reactor **3** and configured to receive the synthesis solution: an aqueous solution comprising urea, ammonium carbamate, and free ammonia; and to fully or partially remove the ammonium carbamate and free ammonia comprised in the synthesis solution. The synthesis reactor **3** is connected to a carbon dioxide compressor **4,** which is configured to provide a stream of gaseous carbon dioxide under high pressure, for example from 130 to 200 bar. The synthesis reactor **3** is also connected to an ammonia pump **4,** which is configured to provide ammonia to the synthesis reactor **3.**

Figure 2 represents the synthesis section of another embodiment of a urea-producing plant according to the present disclosure. Like the urea-producing plant of Figure 1, this urea-producing plant comprises a boiling section **1** comprising one or more electric boilers, configured to produce high-pressure steam and to deliver steam to the HP stripper **2.** The plant also comprises a synthesis reactor **3,** a carbon dioxide compressor **4,** and an ammonia pump **5.** In addition, the plant comprises a HP carbamate condenser **7,** and an ejector **6.** The HP carbamate condenser **7** is configured to receive from the HP stripper a gaseous stream containing ammonia, carbon dioxide and water, and to condense this gaseous stream into an aqueous solution comprising ammonium carbamate. The ejector **6** is connected to the HP carbamate condenser **7** and the ammonia pump **5 and** is configured to use the ammonia from the pump **5** as motive fluid to inject the aqueous solution from the HP carbamate condenser **7** into the synthesis reactor **3.** The HP stripper **2** is configured to produce an aqueous solution comprising urea, ammonium carbamate and free ammonia, and is connected to a medium-pressure or low-pressure section configured to purify and concentrate the aqueous solution produced by the HP stripper **2.**

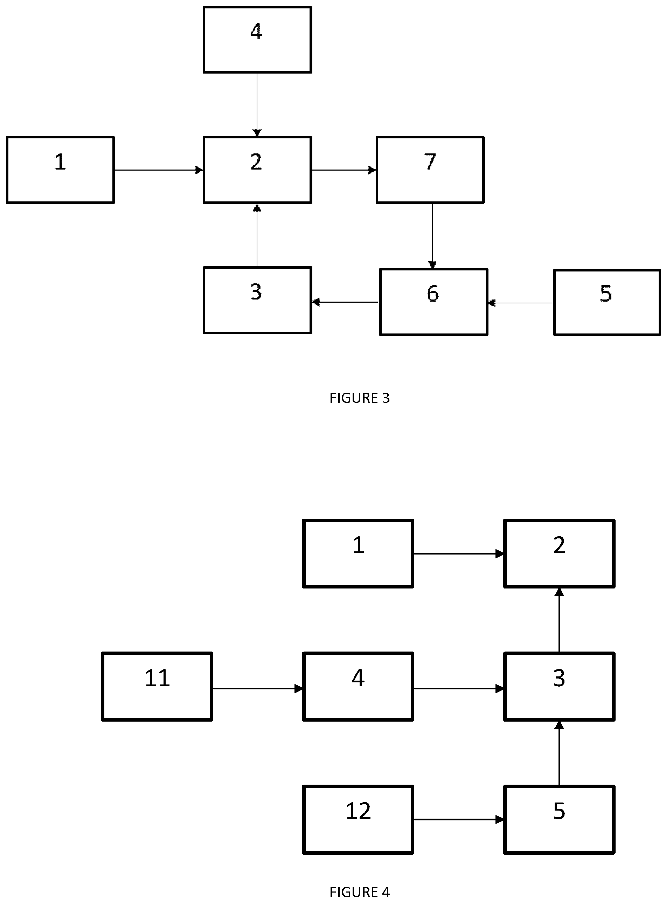

Figure 3 represents the synthesis section of another embodiment of a urea-producing plant according to the present disclosure. Like the urea-producing plant of Figure 2, this urea-producing plant comprises a boiling section **1** comprising one or more electric boilers, configured to produce high-pressure steam and to deliver steam to the HP stripper **2,** a synthesis reactor **3,** a carbon dioxide compressor **4,** an ammonia pump **5,** a HP carbamate condenser **7,** and an ejector **6.** In this embodiment, the carbon dioxide compressor **4** is connected to the HP stripper **2,** such that the carbon dioxide provided by the carbon dioxide compressor **2** is used as a stripping gas in the HP stripper **2.** In operation, the carbon dioxide from the carbon dioxide compressor **2** along with the stripped gases, carbon dioxide and ammonia, and water is directed to the HP carbamate condenser **7,** where an aqueous solution of ammonium carbamate is produced. As in figure 2, the plant comprises an ejector **6,** configured to receive an aqueous solution of ammonium carbamate from the HP carbamate condenser **7** and inject that aqueous solution into the synthesis reactor **3,** using ammonia provided by the ammonia pump **5** as motive fluid.

Figure 4 represents the synthesis section of another embodiment of a urea-producing plant according to the present disclosure. Like the urea-producing plant of Figure 1, this urea-producing plant comprises a boiling section **1,** configured to produce high-pressure steam and to deliver steam to the HP stripper **2** via a first high-pressure steam network. The plant also comprises a synthesis reactor **3,** a carbon dioxide compressor **4,** and an ammonia pump **5.** In addition, the plant comprises two electric motors **11,** and **12.** The first electric motor **11** is connected to the carbon dioxide compressor **4** and configured to provide power to the compressor **4.** The second electric motor **12** is connected to the ammonia pump **5** and configured to provide power to the pump **5.**

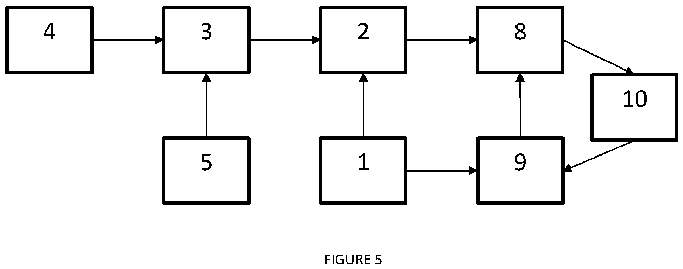

Figure 5 represents a section of another embodiment of a urea-producing plant according to the present disclosure. This urea-producing plant comprises a boiling section **1,** configured to produce high-pressure steam and to deliver steam to the HP stripper **2** via a first high-pressure steam network. The plant also comprises a synthesis reactor **3,** a carbon dioxide compressor **4,** and an ammonia pump **5.** The plant also comprises a low-pressure and/or medium pressure section **8** connected to the HP stripper **2** and configured to receive an aqueous composition comprising urea, ammonium carbamate and free ammonia. The low-pressure and/or medium section **8** is connected to a concentration section **10** configured to transform the aqueous solution from the low-pressure and/or medium-pressure section **8** into a urea melt. The concentration section **10** comprises evaporators that produce an aqueous solution comprising a small amount of urea. The plant comprises a hydrolyzer **9,** which is configured to receive the aqueous solution from the concentration section **10** and remove urea from it. The hydrolyzer **9** performs its task by heating up the aqueous solution to decompose urea back into ammonia and carbon dioxide. The hydrolyzer **9** is connected to the boiling section **1** and configured to receive high-pressure steam from the boiling section **1** in operation. The hydrolyzer 9 is also connected to the low-pressure and/or medium-pressure section **8** such that the gaseous stream produced by the hydrolyzer **9** can be re-introduced in the production process either directly as a gas stream, or it may first go through a condenser to be transformed into an aqueous solution, which is then injected in the low-pressure and/or medium-pressure section **8.**

## Claims

1. A urea-producing plant comprising a first high-pressure steam network, and a device connected to the first high-pressure steam network, **characterized in that** the urea-producing plant further comprises one or more electric boilers, configured to produce high-pressure steam and deliver this high-pressure steam to the first high-pressure steam network.

2. The urea-producing plant according to claim 1, wherein the one or more electric boilers are configured to produce steam at a pressure of 1.0 to 2.5 MPa.

3. The urea-producing plant according claim 1 or 2, wherein the first high-pressure steam network is configured to receive steam at a pressure of from 1.0 to 2.5 MPa.

4. The urea-producing plant according to any one of claims 1 to 3, wherein the device connected to the first high-pressure steam network is a high-pressure stripper or a urea hydrolyzer.

5. The urea-producing plant according to any one of claims 1 to 4, wherein the urea-producing plant comprises a high-pressure stripper and a urea hydrolyzer.

6. The urea-producing plant according to any one of claims 1 to 5, wherein the urea-producing plant comprises a second low-pressure steam network configured to receive steam at a pressure of from 0.1 to 0.7 MPa.

7. The urea-producing plant according to any one of claims 1 to 6, further comprising a device selected from the group consisting of a condenser, a liquid-vapor separator, a carbamate decomposer, and a concentrator.

8. The urea-producing plant according to any one of claims 1 to 7, wherein the urea-producing plant comprises a carbon dioxide compressor and an electric motor configured to provide power to the carbon dioxide compressor.

9. The urea-producing plant according to any one of claims 1 to 8, wherein the urea-producing plant comprises an ammonia pump and an electric motor configured to provide power to the ammonia pump.

10. The urea-producing plant according to any one of claims 1 to 9, further comprising a device selected from the group consisting of a synthesis reactor, and a high-pressure carbamate condenser.

11. The urea-producing plant according to any one of claims 1 to 10, wherein the plant comprises one or more pumps, and wherein said one or more pumps are driven by electric motors.

12. The urea-producing plant according to any one of claims 1 to 11, further comprising a section selected from the group consisting of a synthesis section, a medium-pressure section, a low-pressure section, a concentration section, and a solidification section.

13. A method for producing a urea melt in a urea-producing plant according to any one of claims 1 to 12, wherein the urea-producing plant comprises a first high-pressure steam network, a device connected to the first high-pressure steam network, and one or more electric boilers, the method comprising the steps of:
a) producing high-pressure steam with the one or more electric boilers;
b) directing the high-pressure steam produced in step a) to the first high-pressure steam network;
c) mixing carbon dioxide and ammonia in a reactor, thereby producing a synthesis solution comprising water, urea, ammonium carbamate, and free ammonia;
d) providing high-pressure steam from the first high-pressure steam network and the synthesis solution produced in step c) to a high-pressure stripper, thereby producing an aqueous solution comprising urea and depleted of ammonium carbamate and free ammonia;
e) processing the aqueous solution obtained in step d) to obtain a urea melt.

14. The method of claim 13, wherein the urea melt produced in step e) comprises at least 95.0 weight% of urea.
